# DEMANDE DE BREVET EUROPEEN

(11) **EP 2 713 159 A2**
(43) Date de publication de la demande: **02.04.2014**
(21) Numéro de dépôt: 13186346.6
(22) Date de dépôt: 27.09.2013
(51) Int. Cl.: G01N 33/00

(54) **Procédé de détection d'éléments polluants dans l'air et de pilotage d'un système de décontamination, sonde de détection d'une pollution de l'air et système de traitement d'air comprenant une telle sonde**

(30) Priorité: 27.09.2012 FR 1259125
(71) Demandeur: Arcom, 71530 La Loyere (FR)
(72) Inventeur: Delhomme, Bernard, 42170 Saint Just Saint Rambert (FR); Lapierre, Vincent, 71100 Chalon Sur Saone (FR)
(74) Mandataire: Myon, Gérard Jean-Pierre

(57) **Abrégé**

Ce procédé de d'éléments polluants dans l'air d'un local, notamment de bureau ou d'habitation, et de pilotage d'un système de décontamination, comprend une étape consistant à a) mesurer (100) la teneur en COV de l'air circulant dans le local à l'aide d'un capteur délivrant un paramètre lié à la teneur en éléments polluants. Ce procédé comprend en outre des étapes consistant à b) calculer (101), à des intervalles de temps prédéterminés, la variation temporelle du paramètre représentatif de la quantité d'éléments polluants contenue dans l'air circulant dans le local délivré par le capteur à l'étape a), c) comparer (102) la variation temporelle ou une somme cumulée des variations temporelles calculées à l'étape b) à une valeur seuil, d) si la valeur de la variation temporelle ou de la somme est positive et supérieure à la valeur seui, déclencher (103) un processus de décontamination de l'air circulant dans le local par le système de décontamination.

## Description

L'invention concerne un procédé de détection de polluants dans l'air circulant dans un local, notamment de bureau ou d'habitation, et de pilotage d'un système de décontamination. L'invention concerne également une sonde de détection d'une pollution de l'air, et un système de traitement d'air comprenant une telle sonde.

Des polluants tels que les composés organiques volatiles (ci-après désignés par l'abréviation COV) ou l'ammoniac sont des polluants très répandus dans l'air. En fonction des personnes, l'inhalation de ces produits peut éventuellement déclencher des problèmes de santé, notamment des difficultés respiratoires, voire des dommages neurologiques et/ou cardiovasculaires. On s'efforce donc de détecter ces polluants dans l'air afin d'en réduire la présence, notamment dans les bâtiments, par des systèmes de décontamination chimiques. Il apparaît notamment que les COV sont vingt fois plus présents à l'intérieur des bâtiments qu'à l'extérieur. Le mobilier de bureau, les peintures et les revêtements de sols sont notamment des sources d'émission de COV. L'ammoniac est présent dans l'air à cause de son utilisation dans diverses industries.

Il est connu d'utiliser des capteurs de COV détectant différents types de polluants et permettant de délivrer un paramètre représentatif de la teneur en COV dans l'air. De tels capteurs ne permettant pas de gérer continuellement la qualité de l'air, notamment au niveau d'un système centralisé de traitement d'air. En outre, les capteurs disponibles sur le marché, qui délivrent généralement une tension électrique proportionnelle au niveau de pollution de l'air, ont une précision aléatoire et doivent être souvent recalibrés pour délivrer une mesure précise de la teneur en COV dans l'air. Cette recalibration étant complexe et relativement longue, elle est souvent négligée, ce qui réduit la fiabilité du capteur sur le long terme.

Il est connu également d'utiliser des sondes PID ou à ionisation mises en place dans les flux d'air des réseaux aérauliques. Cependant ces dispositifs ne permettent pas de réguler la qualité de l'air en temps réel.

En outre, ces inconvénients font qu'il est nécessaire de maintenir un fonctionnement permanent des systèmes de décontamination installés dans les locaux. Cela est nocif pour leur fonctionnement et réduit leur durée de vie.

C'est à ces inconvénients qu'entend remédier l'invention en proposant un nouveau procédé de détection d'éléments polluants dans l'air circulant dans un local et de pilotage d'un système de décontamination, permettant de réguler la qualité de l'air en temps réel dans le local en évitant les problèmes liés à la fiabilité et au fonctionnement des capteurs de COV connus de l'état de la technique et en améliorant la durée de vie du système de décontamination.

A cet effet, l'invention concerne un procédé de détection d'éléments polluants dans l'air d'un local, notamment de bureau ou d'habitation, et de pilotage d'un système de décontamination comprenant une étape consistant à
- a) mesurer la teneur en éléments polluants dans l'air circulant dans le local, à l'aide d'un capteur délivrant un paramètre lié à la teneur en éléments polluants.

Ce procédé étant **caractérisé en ce qu'il** comprend en outre des étapes consistant à:
- b) calculer, à des intervalles de temps prédéterminés, la variation temporelle du paramètre représentatif de la teneur en éléments polluants dans l'air circulant dans le local délivré par le capteur à l'étape a) ;
- c) comparer la variation temporelle ou une somme cumulée des variations temporelles calculées à l'étape b) à une valeur seuil;
- d) si la valeur de la variation temporelle ou de la somme est positive et supérieure à la valeur seuil, déclencher un processus de décontamination de l'air circulant dans le local par le système de décontamination.

Grâce à l'invention, en mesurant la variation de la quantité d'éléments polluants dans l'air du local, on obtient une donnée fiable sur la quantité d'éléments polluants dans l'air par rapport à une situation normale, ce qui permet de détecter leur arrivée. En outre, en mesurant les variations temporelles des paramètres délivrées par les capteurs, on s'affranchit des problèmes de fiabilité et de précision de ces capteurs liés à leur calibrage. L'invention permet également de ne déclencher le système de décontamination qu'en cas de nécessité et non de façon permanente. Ceci permet d'augmenter significativement sa durée de vie.

Selon des aspects avantageux mais non obligatoires de l'invention, un tel procédé peut incorporer une ou plusieurs des caractéristiques suivantes, prises selon toute combinaison techniquement admissible :
- Les opérations effectuées aux étapes a), b) et c) sont poursuivies pendant le processus de décontamination initié à l'étape d).
- Le procédé comprend une étape supplémentaire consistant à e) si, pendant le processus de décontamination initié à l'étape d), la valeur de la variation temporelle ou de la somme cumulée est négative et égale, en valeur absolue, à la valeur seuil, arrêter le processus de décontamination.
- Si la somme des variations calculée mesurée pendant le processus de décontamination est inférieure, en valeur absolue, à la valeur seuil, et que le temps écoulé depuis l'enclenchement du processus de décontamination dépasse une valeur prédéterminée, le processus de décontamination est arrêté et une signalisation de défaut de fonctionnement du système de décontamination est activée.
- Si la somme cumulée des variations calculées à l'étape b) reste inférieure à la valeur seuil, l'étape d) est effectuée lorsque le temps écoulé depuis le début des mesures de variation dépasse une valeur prédéterminée.
- Le paramètre mesuré à l'étape a) est une tension électrique proportionnelle à la quantité d'éléments polluants présente dans l'air circulant dans le local.
- A l'étape a), on mesure la teneur en composés organiques volatils (COV), et/ou en ammoniac dans l'air circulant dans le local.

L'invention concerne également une sonde de détection d'une pollution de l'air circulant dans un local, comprenant un capteur délivrant en sortie un paramètre représentatif de la quantité d'éléments polluants contenus dans l'air circulant dans le local. Cette sonde est **caractérisée en ce qu'**elle comporte en outre des moyens de mesure des variations temporelles du paramètre de sortie.

Selon des aspects avantageux mais non obligatoires de l'invention, une telle sonde peut incorporer une ou plusieurs des caractéristiques suivantes, prises dans toute combinaison techniquement admissible :
- Elle comprend en outre des moyens de sommage des variations temporelles des moyens de comparaison des variations temporelles ou de la somme des variations temporelles à une valeur seuil et des moyens de pilotage d'un système de décontamination.
- Les moyens de mesure des variations temporelles du paramètre de sortie, les moyens de sommage, les moyens de comparaison et les moyens de pilotage comprennent un micro contrôleur.
- Le capteur de pollution délivre une tension électrique proportionnelle à la quantité d'éléments polluants contenus dans l'air circulant dans le local.
- Le capteur délivre en sortie un paramètre représentatif de la teneur en composés organiques volatils (COV) et/ou en ammoniac dans l'air circulant dans le local.

L'invention concerne également un système de traitement de l'air circulant dans un local, notamment de bureau ou d'habitation, pour en réduire la teneur en éléments polluants, comprenant un système de ventilation, au moins une entrée d'air dans le système de ventilation, une sortie d'air décontaminé du système de ventilation et un système de décontamination de l'air passant dans le système de ventilation. Ce système de traitement d'air est **caractérisé en ce qu'il** comporte une sonde de détection d'une pollution de l'air telle que mentionnée ci-dessus, placée en entrée du système de ventilation.

Selon un aspect avantageux, le système de décontamination est apte à éliminer les COV et/ou l'ammoniac contenus dans l'air.

L'invention sera mieux comprise et d'autres avantages de celle-ci apparaîtront plus clairement à la lumière de la description qui va suivre d'un mode de réalisation d'un procédé de détection et de pilotage, d'une sonde de détection et d'un système de traitement d'air conformes à son principe, faite en référence aux dessins annexés dans lesquels :
- la figure 1 est un schéma fonctionnel d'une sonde de détection conforme à l'invention ;
- la figure 2 est un schéma fonctionnel d'un système de traitement d'air conforme à l'invention ;

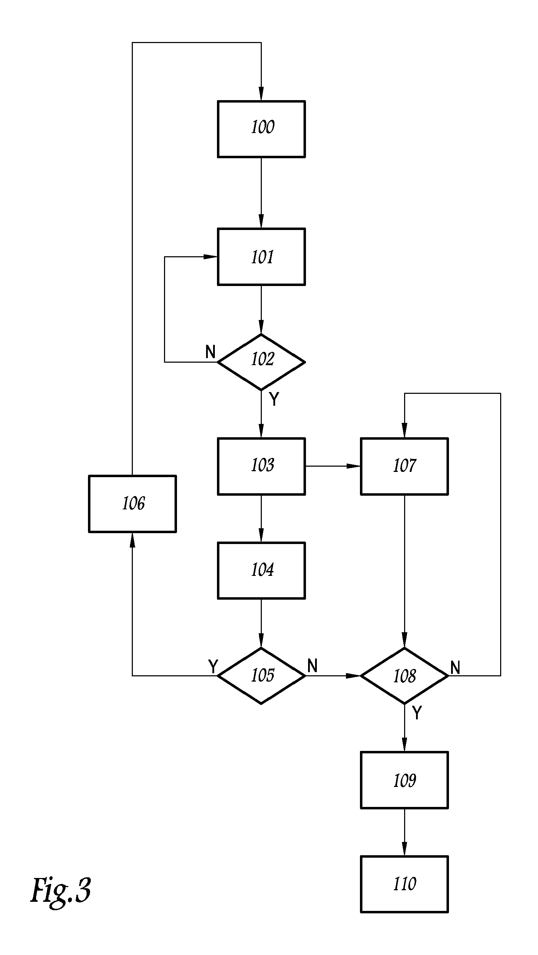

- la figure 3 est un logigramme décrivant les étapes d'un procédé de détection et de pilotage conforme à l'invention ;
- les figures 4 et 5 sont des diagrammes représentant l'évolution dans le temps d'une tension électrique délivrée par un capteur appartenant à une sonde de détection conforme à l'invention.

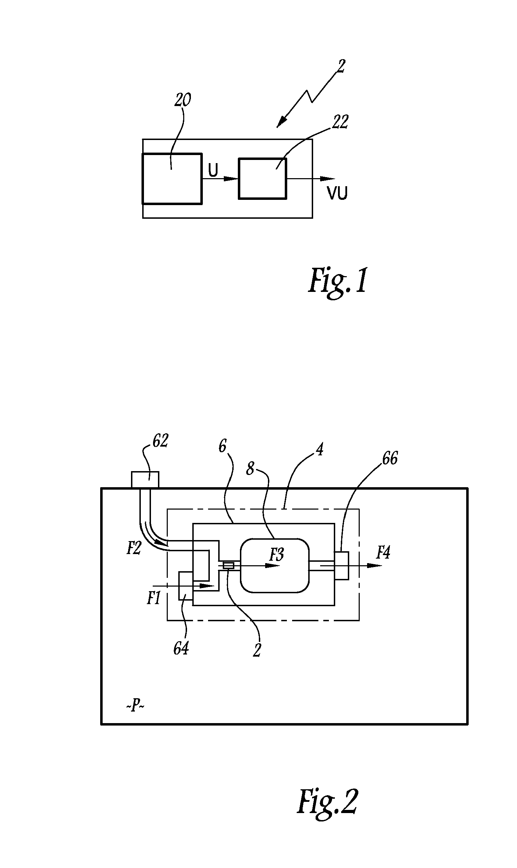

La sonde 2 représentée sur la figure 1 comprend un capteur de COV 20 adapté pour délivrer une tension U, qui est proportionnelle à la quantité de polluants dans l'air.

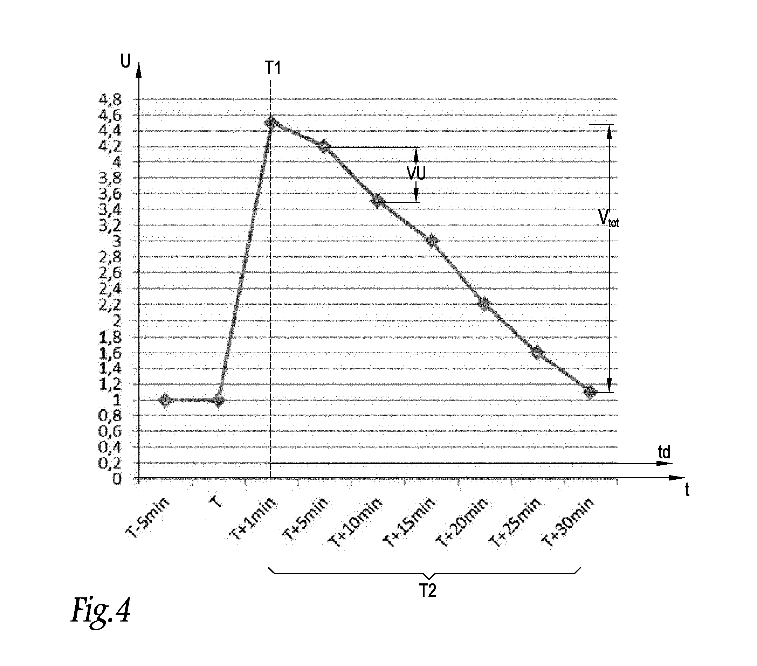

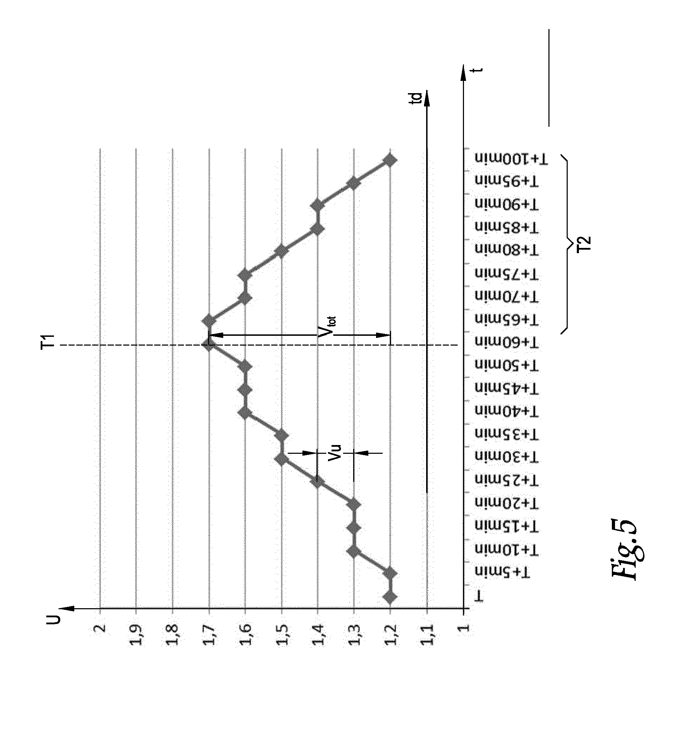

Les figures 4 et 5 représentent la tension délivrée par le capteur 20 en fonction du temps. La sonde 2 comporte en outre un micro contrôleur 22 qui reçoit la tension U et en calcule la dérivée, de manière à déterminer ses variations temporelles VU. Le micro contrôleur 22 génère un signal de sortie de la sonde 2 sous la forme d'une valeur positive ou négative, selon que la variation de la tension U est une augmentation ou une diminution.

En variante, le capteur 20 peut également être adapté pour détecter l'ammoniac présent dans l'air et délivrer une tension électrique représentative de la quantité d'ammoniac présent dans l'air.

Un système de traitement de l'air 4 est représenté sur la figure 2. Le système 4 est installé dans une pièce P et vise à insuffler de l'air décontaminé dans la pièce P. Le système de traitement d'air 4 comporte un système de ventilation 6. Le système de ventilation 6 aspire de l'air potentiellement pollué représenté par la flèche F1 dans la pièce P par une bouche d'aspiration d'air 64. Le système de ventilation 6 aspire également de l'air frais représenté par la flèche F2, provenant par exemple de l'extérieur d'un bâtiment dans lequel la pièce P se trouve, par une conduite d'air frais 62 reliée à une bouche d'aspiration d'air extérieur.

En entrée du système de ventilation 6, une sonde de détection 2 est disposée de manière à mesurer en permanence la quantité de polluants dans le flux d'air arrivant dans le système de ventilation 6 représenté par la flèche F3. La sonde 2 est de préférence mise en place entre la bouche 64 et le système de ventilation 6, l'air se trouvant dans la pièce P étant le plus susceptible d'être pollué par les COV.

La sortie de la sonde 2 est reliée à un système 8 de décontamination de l'air. Le système 8 réduit la pollution de l'air par les COV en séparant les molécules de composés polluants pour obtenir de l'eau et du dioxyde de carbone.

Le système 8 peut également être adapté pour réduire la pollution de l'air par l'ammoniac en séparant les molécules d'ammoniac selon un procédé chimique similaire.

Sur la figure 2, le système de décontamination 8 est intégré au système de ventilation 6. En variante, le système de décontamination 8 peut être indépendant du système de ventilation 6. Le système de décontamination 8 peut par exemple faire partie d'un dispositif distinct, intégrant la sonde 2, qui est ensuite connecté à un système de ventilation similaire au système 6.

En sortie de système de ventilation 6, de l'air décontaminé représenté par la flèche F4 est insufflé dans la pièce P par une bouche 66 de diffusion d'air décontaminé. L'air décontaminé peut éventuellement être mélangé à de l'air frais traité par le système 4 par exemple, pour en réguler sa température.

Le système de traitement d'air 4 fonctionne de la façon suivante. Lors d'une étape initiale 100, le système 4, et plus particulièrement la sonde de détection 2, est mis sous tension. La sonde 2 initie la mesure de la teneur en COV de l'air circulant dans la pièce P. Le capteur 20 délivre à des intervalles de temps réguliers une tension électrique U proportionnelle à la concentration de COV dans l'air circulant dans la pièce P.

Les profils de tension U sont alors transmis par signaux électriques au microcontrôleur. Le profil de tension de la figure 4 représente la réponse en tension U du capteur 20 à la pulvérisation, à l'instant T, d'un désodorisant dans la pièce P. Le profil de tension de la figure 5 montre la réponse en tension U du capteur 20 à la pollution créée par la présence de trois personnes dans la pièce P.

Dans une étape 101, le micro contrôleur 22 de la sonde 2 intègre la tension U résultant des mesures du capteur 20 pour en déduire les variations temporelles VU. Les calculs de variations sont répétés à intervalles de temps réguliers. Cet intervalle de temps peut, par exemple, être compris entre 20 minutes et 1 heure. Ceci permet d'observer la stagnation, l'augmentation ou la diminution du taux de COV dans l'air, en observant l'augmentation, la stagnation ou la diminution des tensions électriques U délivrées par le capteur 20.

La sonde 2, par l'intermédiaire du micro contrôleur 22, délivre, par exemple grâce à des signaux électriques, les variations VU de tension U sous la forme de valeurs qui peuvent être positives ou négatives. Si la valeur de VU est positive, on en déduit que la tension électrique U a augmenté, sur le dernier intervalle de temps, traduisant une augmentation de la concentration d'éléments polluants dans l'air circulant dans la pièce P, comme cela est le cas entre les instants T et T+1 min sur la figure 4. Si la valeur VU est négative, on en conclut que la tension U a diminué, traduisant la diminution de la concentration en agents polluants dans l'air.

Si la valeur VU est voisine de 0, on en conclut que la concentration en COV dans l'air circulant dans la pièce P n'a pas évolué de façon significative dans le dernier intervalle de temps. On considère qu'une valeur de variation VU comprise dans un intervalle de 5 % autour d'une valeur de tension U donnée peut être considérée comme étant négligeable et ne pouvant permettre de conclure à une augmentation significative de la teneur en polluants de l'air.

Dans une étape 102, la somme cumulée Vₜₒₜ des valeurs VU obtenues à l'étape 101 est comparée, à des intervalles de temps régulier, à une valeur seuil Vₘₐₓ, au-delà de laquelle l'augmentation de la tension est représentative de l'apparition d'une pollution importante de l'air qui nécessite une décontamination.

Si la valeur de variation cumulée Vₜₒₜ mesurée est supérieure à Vₘₐₓ, une étape de décontamination 103 débute et le système de décontamination 8 est activé. Ce moment correspond à l'instant T1 sur les figures 4 et 5. Si la valeur Vₜₒₜ est inférieure à Vₘₐₓ, les étapes 101 et 102 continuent à se dérouler de façon normale.

Dans le cas où un processus de décontamination est initié à l'étape 103, les mesures et comparaisons effectuées dans les étapes 101 et 102 se poursuivent dans deux étapes similaires 104 et 105 afin de mesurer l'effet du processus de décontamination sur la teneur en COV de l'air, et de vérifier le bon fonctionnement du système de décontamination 8. Le déroulement du processus de décontamination est repéré par l'intervalle de temps T2 sur les figures 4 et 5. Si les mesures délivrées par le capteur 20 après le début de la phase de décontamination révèlent une variation cumulée Vₜₒₜ négative et égale, en valeur absolue, à la valeur seuil Vₘₐₓ, alors on peut déduire que la teneur en polluants de l'air est revenue au niveau qui était le sien avant que l'augmentation de la teneur en polluants ne s'amorce.

Si la variation cumulée Vₜₒₜ est négative et égale, en valeur absolue, à la valeur Vₘₐₓ, le système de décontamination 8 est désactivé dans une étape 106 et le système 4 retourne aux étapes 101 et 102 pour effectuer les mesures normales de teneur en COV de l'air et comparer les variations V de tension à la valeur seuil Vₘₐₓ.

Lorsque le système de décontamination 8 est activé lors de l'étape 103, un comptage temporel est également activé dans une étape 107, afin de mesurer le temps écoulé depuis le début de la phase de décontamination. On note td le temps écoulé depuis l'activation du système de décontamination 8. Dans une étape 108, on compare le temps td écoulé à une valeur seuil T3. Si au bout de ce temps limite T3 la teneur en polluants de l'air n'est pas revenue à un niveau normal, il est probable qu'un dysfonctionnement du système de décontamination 8 ou de la sonde 2 est survenu. Dans ce cas, le processus de décontamination est arrêté dans une étape 109 et une signalisation de défaut de fonctionnement est activée dans une étape 110. Cela peut être mis en oeuvre par une signalisation visuelle, éventuellement installée sur un boîtier renfermant le système de ventilation 6.

Selon un mode de réalisation de l'invention non représenté, le temps écoulé depuis le début des mesures de variations peut être mesuré afin de mettre en place des phases de décontamination régulières systématiques. Dans ce cas, le système de décontamination 8 est activé lorsque le temps écoulé depuis le début des mesures de variations dépasse une valeur prédéterminée. Cette valeur peut être, par exemple 180 minutes.

Le pilotage du système de décontamination 8 peut être effectué de deux façons différentes. Le micro contrôleur 22 peut être adapté pour élaborer, à partir des mesures de variations VU déterminées sur la base de la tension électrique U délivrée par le capteur 20, des consignes de pilotage à destination du système de décontamination 8. Dans ce cas, le système de décontamination 8 reçoit directement des ordres d'activation ou de désactivation de son fonctionnement. La sonde 2 comprend alors, de préférence dans le microcontrôleur 22, des moyens de sommage des variations temporelles VU pour obtenir la variation cumulée Vₜₒₜ, des moyens de comparaison de Vₜₒₜ avec la valeur seuil Vₘₐₓ, et des moyens de pilotage du système de décontamination 8. Ces moyens de pilotage peuvent notamment comprendre un émetteur de signaux électriques de commande filaire, par radio ou tout autre moyen adapté.

En variante, la sonde 2 peut directement délivrer au système de décontamination 8, par l'intermédiaire du micro contrôleur 22, les variations calculées. Dans ce cas, le système de décontamination 8 interprète les variations grâce à des moyens électroniques intégrés, et est piloté par son propre système de pilotage en fonction des variations VU émises par la sonde 2. Dans ce cas, le système de décontamination 8 comporte par exemple une unité électronique de traitement programmable. Des paramètres tels que les valeurs de Vₘₐₓ et T3 peuvent notamment être programmés par un utilisateur.

Les graphiques représentés sur les figures 4 et 5 sont des exemples de mesures prises par le capteur 20, de temps de mesures et d'instants de mise en fonctionnement du système de décontamination 8. Les valeurs numériques représentées dans ces graphiques sont données à titre d'exemple et ne constituent en aucun cas une limitation de la portée de l'invention.

Les étapes de détection et de pilotage du système de décontamination 8 décrites ci-dessus s'appliquent également à la détection et à l'élimination d'une pollution par l'ammoniac, dans le cas où la sonde 2 et le système de décontamination 8 sont aptes à détecter et éliminer l'ammoniac.

La détection et l'élimination des COV et de l'ammoniac par la sonde 2 et le système 8 peuvent également être réalisées simultanément dans le cadre de l'invention.

Selon une variante de l'invention, la méthode de détection peut également permettre de comparer directement les variations temporelles VU calculées aux étapes 101 et 104 à la valeur seuil Vmax. Cette variante peut être combinée à tous les modes de réalisation de l'invention.

## Revendications

1. Procédé de détection d'éléments polluants dans l'air d'un local (P), notamment de bureau ou d'habitation, et de pilotage d'un système de décontamination (8), comprenant une étape consistant à
- a) mesurer (100) la teneur en éléments polluants dans l'air circulant dans le local (P), à l'aide d'un capteur (20) délivrant un paramètre (U) lié à la teneur en éléments polluants,
ce procédé étant **caractérisé en ce qu'il** comprend en outre des étapes consistant à :
- b) calculer (101) à des intervalles de temps prédéterminés, la variation temporelle (VU) du paramètre (U) représentatif de la quantité d'éléments polluants contenue dans l'air circulant dans le local délivré par le capteur (20) à l'étape a) ;
- c) comparer (102) la variation temporelle (VU) ou une somme (Vₜₒₜ) cumulée des variations temporelles (VU) calculées à l'étape b) à une valeur seuil (Vₘₐₓ) ;
- d) si la valeur de la variation temporelle (VU) ou de la somme (Vₜₒₜ) est positive et supérieure à la valeur seuil (Vₘₐₓ), déclencher (103) un processus de décontamination de l'air circulant dans le local par le système de décontamination (8).

2. Procédé selon la revendication 1, **caractérisé en ce que** les opérations (101, 102) effectuées aux étapes a), b) et c) sont poursuivies (104, 105) pendant le processus de décontamination (103) initié à l'étape d).

3. Procédé selon la revendication 2, **caractérisé en ce qu'il** comprend une étape supplémentaire consistant à :
- e) si, pendant le processus de décontamination (103) initié à l'étape d), la valeur de la variation temporelle (VU) ou de la somme (Vₜₒₜ) cumulée est négative et égale, en valeur absolue, à la valeur seuil (Vₘₐₓ), arrêter (106) le processus de décontamination.

4. Procédé selon l'une des revendications 2 et 3, **caractérisé en ce que** si la somme (Vₜₒₜ) des variations (VU) calculée pendant le processus de décontamination est inférieure, en valeur absolue, à la valeur seuil (Vₘₐₓ), et que le temps (td) écoulé depuis l'enclenchement (103) du processus de décontamination dépasse une valeur prédéterminée (T3), le processus de décontamination est arrêté (109) et une signalisation de défaut de fonctionnement du système (8) de décontamination est activée (110).

5. Procédé selon l'une des revendications précédentes, caractérisé en ce si la variation temporelle (VU) ou de la somme (Vₜₒₜ) cumulée des variations (VU) calculées à l'étape b) reste inférieure à la valeur seuil (Vₘₐₓ), l'étape d) est effectuée lorsque le temps écoulé depuis le début des mesures de variation dépasse une valeur prédéterminée.

6. Procédé selon l'une des revendications précédentes, **caractérisé en ce que** le paramètre mesuré à l'étape a) est une tension électrique (U) proportionnelle à la quantité d'éléments polluants présente dans l'air circulant dans le local (P).

7. Procédé selon l'une des revendications précédentes, **caractérisé en ce qu'à** l'étape a), on mesure la teneur en composés organiques volatils (COV), et/ou en ammoniac dans l'air circulant dans le local (P).

8. Sonde (2) de détection d'une pollution de l'air circulant dans un local (P), comprenant un capteur (20) délivrant en sortie un paramètre représentatif (U) de la quantité d'éléments polluants contenus dans l'air circulant dans le local, **caractérisée en ce qu'**elle comporte en outre des moyens (22) de calcul des variations temporelles (VU) du paramètre de sortie (U).

9. Sonde selon la revendication 8, **caractérisé en ce qu'**elle comprend en outre des moyens (22) de sommage des variation temporelles (VU), des moyens de comparaison des variations temporelles (VU) ou de la somme (Vₜₒₜ) des variations temporelles (VU) à une valeur seuil (Vₘₐₓ) et des moyens (22) de pilotage d'un système de décontamination (8).

10. Sonde selon la revendication 9, **caractérisée en ce que** les moyens de calcul des variations temporelles (VU) du paramètre de sortie (U) du capteur (20), les moyens de sommage, les moyens de comparaison et les moyens de pilotage comprennent un microcontrôleur (22).

11. Sonde selon l'une des revendications 8 à 10, **caractérisée en ce que** le capteur (20) délivre une tension électrique (U) proportionnelle à la quantité d'éléments polluants contenue dans l'air circulant dans le local (P).

12. Sonde selon l'une des revendications 8 à 11, **caractérisée en ce que** le capteur (20) délivre en sortie un paramètre (U) représentatif de la teneur en composés organiques volatils (COV) et/ou en ammoniac dans l'air circulant dans le local (P).

13. Système de traitement (4) de l'air circulant dans un local (P), notamment de bureau ou d'habitation, pour en réduire la teneur en éléments polluants, comprenant un système de ventilation (6), au moins une entrée d'air (62, 64) dans le système de ventilation (6), une sortie (66) d'air décontaminé du système de ventilation (6) et un système (8) de décontamination de l'air passant dans le système de ventilation (6), **caractérisé en ce qu'il** comporte une sonde de détection (2) d'une pollution de l'air selon l'une des revendications 8 à 12, placée en entrée du système de ventilation (6).

14. Système selon la revendication 13, **caractérisé en ce que** le système (8) de décontamination est apte à éliminer les COV et/ou l'ammoniac contenu dans l'air.
